# FASCICULE DE BREVET EUROPEEN

(11) **EP 3 534 984 B1**
(45) Date de publication et mention de la délivrance du brevet: **17.03.2021**
(21) Numéro de dépôt: 17836067.3
(22) Date de dépôt: 03.11.2017
(51) Int. Cl.: A61M 1/34

(54) **DISPOSITIF PORTABLE D'ULTRAFILTRATION**
TRAGBARE ULTRAFILTRATIONSVORRICHTUNG
PORTABLE ULTRAFILTER DEVICE

(30) Priorité: 07.11.2016 MA 39440
(43) Date de publication de la demande: 11.09.2019
(73) Titulaire: Université Hassan II de Casablanca, Casablanca (MA)
(72) Inventeur: ZAMD, Mohamed, Casablanca (MA); RAMDANI, Benyounes, Casablanca (MA); AIT TALEB, Abdellah, Casablanca (MA); BOUALAM, Abdellah, Casablanca (MA)
(74) Mandataire: Cabinet Chaillot
(86) Numéro de dépôt international: PCT/MA2017/000022
(87) Numéro de publication internationale: WO 2018/084690

(56) Documents cités:
- EP-A2- 1 666 078
- WO-A1-95/29731
- US-A- 4 269 708
- US-A1- 2004 254 514
- US-A1- 2014 091 018
- US-A1- 2014 231 350
- US-B2- 8 211 047

## Description

La présente invention concerne un dispositif portable pour ultrafiltration.

L'excès de liquide dans le compartiment extracellulaire est une situation fréquente dans le domaine médicale. Elle touche divers types de patients et conditionne le pronostic vital à cours et à long terme. Les patients concernés par cette situation sont ceux ayant une défaillance rénale (aiguë ou chronique) et/ou une défaillance cardiaque. Lors de cette dernière, surtout dans les stades avancés, une défaillance rénale est souvent associée rendant l'usage des diurétiques (médicaments destinés à évacuer le surplus de liquide par le rein) peu efficace voire totalement inefficace. Dans toutes ces situations, le recours à une extraction de liquide directement du sang devient nécessaire et urgente.

Actuellement, cette extraction, autrement appelée ultrafiltration, se fait à l'aide de machines de grande taille en milieu hospitalier et nécessitent l'immobilisation du patient durant toute la durée de l'intervention (qui peut aller jusqu'à 4 heures). Plusieurs tentatives de miniaturisation des machines d'ultrafiltration ont été effectuées sans pouvoir aboutir à un dispositif assez sûr et efficace pour être utilisé chez les humains à grande échelle.

Nous avons publié un premier brevet dans ce domaine le 12 mai 2016 sous le numéro WO2016/072826. Les essais sur prototype que nous avons effectué pour sa validation ont objectivé une efficacité d'ultrafiltration très importante (extraction du tiers du volume d'une poche de sang en moins d'une demi-heure). Par ailleurs, et de part sa conception, il nous a été impossible de contrôler le volume extrait : une fois le processus enclenché, l'ultrafiltration se poursuit jusqu'à remplissage de l'enveloppe plastique entourant Γ hémofiltre. Par extrapolation sur un système vivant, une perte aussi rapide de liquide sans moyens de contrôle aboutirait à un état de choc irréversible.

Le dispositif selon l'invention permet, de résoudre ce problème de sécurité tout en préservant le caractère portable et la simplicité d'utilisation du dispositif.

US8211047B2 divulgue un dispositif d'ultrafiltration selon le préambule de la revendication 1.

Le dispositif selon l'invention comporte en effet selon une première caractéristique un circuit sanguin constitué d'un hémofiltre relié de part et d'autre à des tubulures plastiques appelées lignes artérielles et veineuses. La ligne artérielle relie l'abord vasculaire (une fistule artérioveineuse ou un cathéter veineux double lumière) à l'hémofiltre et traverse une pompe péristaltique à galets destinée à véhiculer le sang dans le circuit sanguin. La ligne veineuse fait suite à l'hémofiltre et passe par un bloc électronique de sécurité comportant un détecteur de pression veineuse et un détecteur d'air (permettant de lutter contre le risque d'embolie gazeuse) avant de rejoindre l'abord vasculaire. Sur l'un des embouts dialysat de l'hémofiltre est monté un détecteur de pression d'ultrafiltration (qui mesure la pression dans le compartiment qui entoure les fibres creuses de l'hémofiltre). Sur l'autre embout est montée une tubulure qui relie le dit embout à un soufflet aspiratif et passe à travers une électrovanne. La pompe péristaltique à galet, le bloc électronique de sécurité, le détecteur de pression d'ultrafiltration et l'électrovanne sont reliés, contrôlés et gérés par une unité centrale intelligente alimenté par une batterie rechargeable.

Le fonctionnement du dispositif est comme suit : le sang du patient recueilli à partir de l'abord vasculaire et circule sous l'effet de la pompe dans la ligne artérielle puis passe à travers l'hémofiltre pour revenir via la ligne veineuse vers l'abord vasculaire. La pression négative générée par le soufflet aspiratif est transmise à travers la tubulure de raccordement et provoque l'ultrafiltration du sang à travers les fibres de l'hémofiltre. Ainsi du liquide s'accumule dans l'espace entourant les fibres jusqu'au remplissage complet de la coque externe de l'hémofiltre puis, par la tubulure de raccordement, s'accumule dans le soufflet aspiratif jusqu'à remplissage complet de celui-ci. Au fur et à mesure du remplissage du soufflet aspiratif la pression négative qu'il exerce s'atténue jusqu'à devenir nulle puis s'accroit lentement jusqu'à atteindre le niveau de la pression régnant dans le sang contenu dans les fibres. A ce moment-là, l'ultrafiltration s'arrête et l'unité centrale indique la fin du traitement à l'utilisateur par une alarme visuelle et sonore. Le sang est restitué au patient par l'infusion d'une solution physiologique isotonique. L'hémofiltre et le soufflet aspiratif sont alors retirés du dispositif. L'unité centrale intelligente contrôle la marche, l'arrêt et la vitesse de rotation de la pompe à sang, permet la détection de l'air dans la ligne veineuse et enregistre en continu les pressions qui lui proviennent des détecteurs de pression (veineuse et d'ultrafiltration). Ces deux dernières mesures combinées au coefficient d'ultrafiltration de l'hémofiltre (introduit dans la mémoire de l'unité centrale intelligente au début de chaque traitement) permettent à l'unité centrale intelligente de calculer en temps réel la quantité de liquide extraite et de détecter la fin du traitement lorsque les pressions veineuses et d'ultrafiltration s'égalisent. En cas d'intolérance ou de chute de pression artérielle, il est possible à tout moment d'arrêter le processus d'ultrafiltration par une commande au niveau de l'unité centrale intelligente qui active l'électrovanne montée sur la tubulure reliant l'embout dialysat de l'hémofiltre et le soufflet aspiratif. Ainsi, la transmission de la pression négative est interrompue et l'ultrafiltration s'arrête immédiatement. Le volume de liquide extrait est celui du soufflet aspiratif utilisé (variable en fonction des besoins du patient) et il est possible, si un volume supplémentaire est nécessaire, de changer de soufflet aspiratif en fin de traitement par simple activation de l'électrovanne, retrait du soufflet aspiratif plein et installation d'un nouveau sous vide.

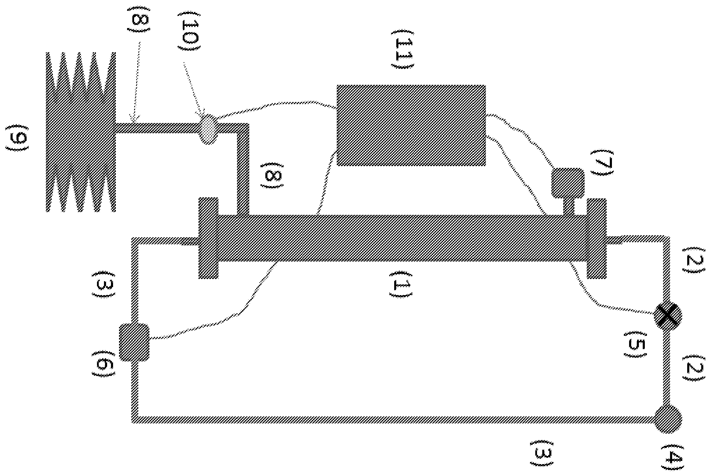

Le dessin annexé illustre l'invention : la figure 1 représente un schéma du dispositif de l'invention.

En référence à ce dessin, le dispositif est constitué d'un circuit sanguin fait d'un hémofiltre (1) relié de part et d'autre à des tubulures plastiques appelées lignes artérielles (2) et veineuses (3). La ligne artérielle (2) relie l'abord vasculaire (4) (une fistule artérioveineuse ou un cathéter veineux double lumière) à l'hémofiltre (1) et traverse une pompe péristaltique (5) à galets destinée à véhiculer le sang dans le circuit sanguin. La ligne veineuse (3) fait suite à l'hémofiltre (1) et passe par un bloc électronique de sécurité (6) comportant un détecteur de pression veineuse et un détecteur d'air avant de rejoindre l'abord vasculaire (4). Sur l'un des embouts dialysat de l'hémofiltre est monté un détecteur de pression d'ultrafiltration (7) (qui mesure la pression dans le compartiment qui entoure les fibres creuses de l'hémofiltre). Sur l'autre embout est montée une tubulure (8) qui relie le dit embout à un soufflet aspiratif (9) et passe à travers une électrovanne (10). La pompe péristaltique à galet (5), le bloc électronique de sécurité (6), le détecteur de pression d'ultrafiltration (7) et l'électrovanne (10) sont reliés et contrôlés par une unité centrale intelligente (11) alimenté par une batterie rechargeable.

Le sang du patient est recueilli à partir de l'abord vasculaire (4) et circule sous l'effet de la pompe (5) dans la ligne artérielle (2) puis passe à travers l'hémofiltre (1) pour revenir via la ligne veineuse (3) vers l'abord vasculaire (4). Une injection d'anticoagulation est nécessaire pour maintenir la fluidité du sang dans le circuit sanguin. La pression négative générée par le soufflet aspiratif (9) est transmise à travers la tubulure (8) de raccordement et provoque l'ultrafiltration du sang à travers les fibres de l'hémofiltre (1). Ainsi du liquide s'accumule dans l'espace entourant les fibres jusqu'au remplissage complet de la coque externe de l'hémofiltre (1) puis, par la tubulure de raccordement (8), s'accumule dans le soufflet aspiratif (9) jusqu'à remplissage complet de celui-ci. Au fur et à mesure du remplissage du soufflet aspiratif (9) la pression négative qu'il exerce s'atténue jusqu'à devenir nulle puis s'accroit lentement jusqu'à atteindre le niveau de la pression régnant dans le sang contenu dans les fibres. A ce moment-là, l'ultrafiltration s'arrête et l'unité centrale (11) indique la fin du traitement à l'utilisateur par une alarme visuelle et sonore. Le sang est restitué au patient par l'infusion d'une solution physiologique isotonique. L'hémofiltre (1) et le soufflet aspiratif (9) sont alors retirés du dispositif. L'unité centrale intelligente (11) contrôle la marche, l'arrêt et la vitesse de rotation de la pompe à sang (5), permet la détection de l'air dans la ligne veineuse (3) et enregistre en continu les pressions qui lui proviennent des détecteurs de pression veineuse provenant du bloc électronique de sécurité (6) et d'ultrafiltration provenant du détecteur (7) placé sur l'embout dialysat de l'hémofiltre). Ces deux dernières mesures combinées au coefficient d'ultrafiltration de l'hémofiltre (1) (introduit dans la mémoire de l'unité centrale intelligente (11) au début de chaque traitement) permettent à l'unité centrale intelligente (11) de calculer en temps réel la quantité de liquide extraite et de détecter la fin du traitement lorsque les pressions veineuses et d'ultrafiltration s'égalisent. En cas d'intolérance ou de chute de pression artérielle, il est possible à tout moment d'arrêter le processus d'ultrafiltration par une commande au niveau de l'unité centrale intelligente (11) qui active l'électrovanne (10) montée sur la tubulure (8) reliant l'embout dialysat de l'hémofiltre (1) et le soufflet aspiratif (9). Ainsi, la transmission de la pression négative est interrompue et l'ultrafiltration s'arrête immédiatement. Le volume de liquide extrait est celui du soufflet aspiratif (9) utilisé (variable en fonction des besoins du patient) et il est possible, si un volume supplémentaire est nécessaire, de changer de soufflet aspiratif (9) en fin de traitement par simple activation de l'électrovanne (10), retrait du soufflet aspiratif (9) plein et installation d'un nouveau sous vide.

La présente invention n'est nullement limitée aux modes de réalisation décrits et représentés mais l'homme du métier saura y apporter toute variante conforme à son esprit.

Les avantages escomptés de la présente invention sont nombreux. La facilité de sa fabrication et le coût modeste de ses composants en font un outil bon marché pour traiter une affection extrêmement fréquente et au pronostic souvent fâcheux. Les coûts actuels de la surcharge hydro-sodée menacent les budgets de santé de plusieurs états. Elle pourrait constituer une réelle alternative dans les zones à moindre revenu surtout dans le traitement de l'insuffisance rénale aiguë compliquée de surcharge hydro-sodée. La portabilité de l'appareil permet de libérer les patients atteints de défaillance cardiaque et/ou rénale des machines d'ultrafiltration et des hospitalisations itératives qui font souvent suite à des accidents de surcharge. Il pourrait leur permettre ainsi une meilleure insertion socioprofessionnelle. Le caractère variable de l'intensité de l'extraction de liquide permet de réduire l'impact sur le coeur : l'extraction est maximale au départ lorsque le volume sanguin est important et diminue de façon exponentielle au fur et à mesure de du remplissage du soufflet aspiratif et de la diminution de la valeur absolue de la pression négative. Par ailleurs, le volume du soufflet aspiratif étant fixé à l'avance aucun dépassement d'objectif n'est possible. Un usage quotidien et régulier de ce dispositif pourrait améliorer le pronostic cardiovasculaire toujours fâcheux des patients souffrant de surcharge hydro-sodée. La dimension écologique de ce procédé est indéniable puisqu'il permet des économies très importantes en matière d'eau, d'électricité et de production de déchets à fort risque infectieux.

## Revendications

1. Dispositif portable d'ultrafiltration dans lequel un soufflet aspiratif (9) est relié par f une tubulure (8) passant à travers une électrovanne (10) à un embout dialysat d'un hémofiltre (1) intégré dans un circuit sanguin fait lui-même de lignes artérielles (2) émanant d'un abord vasculaire (4) et passant à travers une pompe péristaltique (5) et de lignes veineuses (3) et rejoignant l'abord vasculaire (4), **caractérisé par le fait que** l'hémofiltre (1) a un autre embout dialysat qui est relié à un détecteur de pression d'ultrafiltration (7), les lignes veineuses (3) passant à travers un bloc électronique de sécurité (6).

2. Dispositif portable d'ultrafiltration selon la revendication 1 **caractérisé en ce que** la pompe péristaltique (5), le bloc électronique de sécurité (6), le détecteur de pression d'ultrafiltration (7) et l'électrovanne (10) sont reliés et contrôlés par une unité centrale intelligente (11) alimentée par une batterie rechargeable.

3. Dispositif portable d'ultrafiltration selon la revendication 1 et 2 **caractérisé en ce que** le bloc électronique de sécurité (6) est constitué d'un détecteur de pression veineuse qui transmet en temps réel le niveau de pression dans les fibres creuses de l'hémofiltre (1) à l'unité centrale intelligente (11) et d'un détecteur d'air sur la ligne veineuse.

4. Dispositif portable d'ultrafiltration selon les revendications précédentes **caractérisé en ce que** l'unité centrale intelligente (11) calcule en temps réel la quantité de liquide extraite en se basant sur les données transmises par le détecteur de pression veineuse du bloc électronique de sécurité (6) et par le détecteur de pression d'ultrafiltration (7).

5. Dispositif portable d'ultrafiltration selon les revendications précédentes **caractérisé en ce que** l'unité centrale intelligente (11) commande l'électrovanne (10) pour arrêter l'ultrafiltration à tout moment et à la requête de l'utilisateur.

6. Dispositif portable d'ultrafiltration selon les revendications précédentes **caractérisé en ce que** l'unité centrale intelligente (11) avise l'utilisateur du moment de la fin du traitement par une alarme visuelle et sonore.

## Patentansprüche

1. Tragbare Ultrafiltrationsvorrichtung, wobei ein Ansaugbalg (9) durch ein Rohr (8) verbunden ist, das durch ein Magnetventil (10) zu einem Dialysatanschluss eines Hämofilters (1) verläuft, der in einen Blutkreislauf integriert ist, der selbst aus arteriellen Leitungen (2) besteht, die von einem Gefäßzugang (4) abgehen und durch eine Peristaltikpumpe (5) verlaufen, und aus venösen Leitungen (3) und den Gefäßzugang (4) erreichen, **dadurch gekennzeichnet, dass** der Hämofilter (1) einen anderen Dialysatanschluss hat, der mit einem Ultrafiltrations-Druckdetektor (7) verbunden ist, wobei die venösen Leitungen (3) durch einen elektronischen Sicherheitsblock (6) verlaufen.

2. Tragbare Ultrafiltrationsvorrichtung nach Anspruch 1, **dadurch gekennzeichnet, dass** die Peristaltikpumpe (5), der elektronische Sicherheitsblock (6), der Ultrafiltrations-Druckdetektor (7) und das Magnetventil (10) durch eine intelligente Zentraleinheit (11) verbunden sind und gesteuert werden, die von einer aufladbaren Batterie versorgt wird.

3. Tragbare Ultrafiltrationsvorrichtung nach Anspruch 1 und 2, **dadurch gekennzeichnet, dass** der elektronische Sicherheitsblock (6) aus einem Detektor des venösen Drucks besteht, der in Echtzeit das Druckniveau in den Hohlfasern des Hämofilters (1) an die intelligente Zentraleinheit (11) überträgt, und einem Luftdetektor auf der venösen Leitung.

4. Tragbare Ultrafiltrationsvorrichtung nach den vorangehenden Ansprüchen, **dadurch gekennzeichnet, dass** die intelligente Zentraleinheit (11) in Echtzeit die entnommene Flüssigkeitsmenge berechnet, indem sie sich auf die Daten stützt, die von dem Detektor des venösen Drucks des elektronischen Sicherheitsblocks (6) und von dem Ultrafiltrations-Druckdetektor (7) übermittelt werden.

5. Tragbare Ultrafiltrationsvorrichtung nach den vorangehenden Ansprüchen, **dadurch gekennzeichnet, dass** die intelligente Zentraleinheit (11) das Magnetventil (10) steuert, um die Ultrafiltration jederzeit und auf Anforderung des Benutzers anzuhalten.

6. Tragbare Ultrafiltrationsvorrichtung nach den vorangehenden Ansprüchen, **dadurch gekennzeichnet, dass** die intelligente Zentraleinheit (11) den Benutzer über den Moment des Endes der Behandlung durch einen visuellen und akustischen Alarm benachrichtigt.

## Claims

1. A portable ultrafiltration device in which a suction bellows (9) is connected by a tube (8) passing through a solenoid valve (10) to a first dialysate connector of a hemofilter (1) incorporated into a blood circuit itself consisting of arterial lines (2) extending from a vascular access (4) and passing through a peristaltic pump (5) and of venous lines (3), and connecting to the vascular access (4), **characterised in that** the hemofilter (1) has another dialysate connector that is connected to an ultrafiltration pressure sensor (7), the venous lines (3) passing through an electronic safety block (6).

2. The portable ultrafiltration device according to claim 1, **characterised in that** the peristaltic pump (5), the electronic safety block (6), the ultrafiltration pressure sensor (7) and the solenoid valve (10) are connected and controlled by an intelligent central unit (11) powered by a rechargeable battery.

3. The portable ultrafiltration device according to claim 1 and 2, **characterised in that** the electronic safety block (6) consists of a venous pressure sensor which transmits in real time a level of the pressure in the hollow fibers of the hemofilter (1) to the intelligent central unit (11), and of an air sensor on the venous line.

4. The portable ultrafiltration device according to the preceding claims, **characterised in that** the intelligent central unit (11) calculates in real time the extracted amount of liquid on the basis of the data transmitted by the venous pressure sensor of the electronic safety block (6) and by the ultrafiltration pressure sensor (7) .

5. The portable ultrafiltration device according to the preceding claims, **characterised in that** the intelligent central unit (11) controls the solenoid valve (10) to stop the ultrafiltration at any time and at the user request.

6. The portable ultrafiltration device according to the preceding claims, **characterised in that** the intelligent central unit (11) notifies the user of the time of the end of the treatment, with a visual and audible alarm.
